(19) Europäisches Patentamt
European Patent Office
Office européen des brevets

(11) Publication number: **0 181 970**
**B1**

(12) **EUROPEAN PATENT SPECIFICATION**

(45) Date of publication of patent specification: **29.08.90**

(51) Int. Cl.$^5$: **A 61 L 15/16,** A 61 K 9/70

(21) Application number: **84307324.8**

(22) Date of filing: **24.10.84**

(54) **Percutaneously administrable pharmaceutical preparation in the form of an adhesive tape.**

(43) Date of publication of application:
**28.05.86 Bulletin 86/22**

(45) Publication of the grant of the patent:
**29.08.90 Bulletin 90/35**

(84) Designated Contracting States:
**DE FR GB IT NL**

(56) References cited:
**EP-A-0 024 107**
**EP-A-0 054 279**
**EP-A-0 062 682**
**DE-A-2 216 260**
**DE-A-2 636 559**
**FR-A-2 497 457**

**The file contains technical information submitted after the application was filed and not included in this specification**

(73) Proprietor: **SEKISUI KAGAKU KOGYO KABUSHIKI KAISHA**
**4-4 Nishitemma 2-chome**
**Kita-ku Osaka 530 (JP)**

(72) Inventor: **Fukuda, Mutsumi**
**2-8-405 Aoba 3-chome Shimamoto-cho**
**Mishima-gun Osaka (JP)**
Inventor: **Nakagawa, Takashi**
**2-3 Hiyoshidai 1-chome**
**Ohtsu-shi Shiga (JP)**
Inventor: **Kishi, Takaji**
**2-7 Aza-Miyata Koya**
**Itami-shi Hyogo (JP)**
Inventor: **Ando, Michiharu**
**2-2- Hyakuyama Shimamoto-cho**
**Mishima-gun Osaka (JP)**
Inventor: **Yoshioka, Masahachi**
**2-2 Hyakuyama Shimamoto-cho**
**Mishima-gun (JP)**

(74) Representative: **Kosmin, Gerald Emmanuel et al**
**HASELTINE, LAKE & CO. Hazlitt House 28**
**Southampton Buildings Chancery Lane**
**London, WC2A 1AT (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 181 970 B1

**Description**

This invention relates to a percutaneously administrable pharmaceutical preparation in the form of an adhesive tape for use in the treatment of angina pectoris.

Isosorbide dinitrate (1,4:3,6-dianhydro-D-glucitol dinitrate), hereinafter referred to as ISDN, and nitroglycerin (NG) are known as effective medicines for angina pectoris. However, neither of these is effective over a prolonged period of application, nor are they effective in suppressing or preventing attacks of angina pectoris after an initial period of effectiveness. Therefore, a pharmaceutical preparation which would supply ISDN gradually and at a constant rate over a long period of time has been desired.

A percutaneous absorption type pharmaceutical preparation containing ISDN or pentaerythritol tetranitrate (PETN) in the form of an adhesive tape has been proposed in FR—A—2 497 457 wherein a flexible backing is provided with an adhesive coating thereon containing the ISDN or PETN, the adhesive material being composed of a copolymer containing alkyl acrylate or methacrylate in a concentration of at least 50% by weight, a functional polar monomer in a concentration of up to 20% by weight and a vinyl ester monomer in a concentration of up to 40% by weight. Such a percutaneous absorption type pharmaceutical preparation in adhesive tape form has been found to exhibit the following three critical drawbacks.

Firstly, since such a pharmaceutical preparation is administered via the skin having a tissue which serves to prevent foreign substances from entering into the body, it is difficult to administer an amount of active ingredient which is sufficient to take effect immediately. Thus, a large-sized tape must be applied to the skin and/or an absorption promoter must be incorporated into the adhesive material.

Secondly, this pharmaceutical preparation necessarily has a side effect. Due to contact of the adhesive layer with the skin over a given period of time, the normal secretion, metabolism, expansion and regeneration of the skin are prevented and the skin suffers irritation at the edge portion of the tape and/or from the adhesive layer thereof, resulting in a number of red spots, and in extreme cases, incrustations and/or edemata on the skin, which can remain for several days.

Thirdly, a part of the adhesive material unavoidably remains on the skin when the tape is peeled off.

Additionally, a portion of the active ingredient contained in the adhesive material penetrates through the backing layer, and/or crystallises at the interface of the adhesive layer and the backing layer, thereby resulting in reduced pharmaceutical effect.

According to the present invention there is provided a percutaneously administrable pharmaceutical preparation in the form of an adhesive tape comprising a flexible backing which is not permeable to the pharmaceutically active ingredient, and an adhesive coating layer on said flexible backing, the adhesive layer comprising an adhesive material and a pharmaceutically active ingredient compatible with said adhesive material, characterised in that said adhesive material is a copolymer derived from 2-ethylhexyl acrylate (EHA) in a concentration of 45 to 80 mole%, at least one (meth) acrylate monomer in a concentration of from 0 to 35 mole%, the monomer being selected from propyl acrylate, butyl acrylate, hexyl acrylate, 2-ethyl butyl acrylate, heptyl acrylate, octyl acrylate, nonyl acrylate, decyl methacrylate and lauryl methacrylate, and N-vinyl-2-pyrrolidone (VP) in a concentration of from 20 to 55 mole%, with the sum of the mole% of EHA, (meth) acrylate and VP being 100, and said pharmaceutically active ingredient is isosorbide dinitrate (ISDN) which is present in said adhesive material in a concentration of 13 to 30% by weight and which is at least 80% of the saturated solubility concentration of ISDN in said adhesive material.

In a preferred embodiment, the adhesive material is a copolymer derived from EHA in a concentration of 55 to 70 mole% and VP in a concentration of 30 to 45 mole%, and the concentration of ISDN in said adhesive material is 13% to 25% by weight.

The adhesive material may be further derived from at least one (meth)acrylate monomer, in a concentration of 35 mole% or less, which monomer is selected from propyl acrylate, butyl acrylate, hexyl acrylate, 2-ethyl butyl acrylate, heptyl acrylate, octyl acrylate, nonyl acrylate, decyl methacrylate and lauryl methacrylate. Preferably the (meth)acrylate monomer is present in a concentration of 15 mole% or less.

The adhesive material may further be derived from at least one multifunctional monomer in a concentration of preferably from 0.005 to 0.5% by weight of the total amount of monomers to be polymerised. The multifunctional monomer may be selected from di(meth)acrylates, tri(meth)acrylates and tetra-(meth)acrylates.

The pharmaceutical preparation of invention exhibits the following advantages:

(a) there is excellent release of ISDN dissolved in the adhesive material at a high concentration thereby making it possible to attain an effective administration of ISDN per unit area;

(b) the percutaneous administration of ISDN is significantly less irratative to the skin;

(c) there is excellent release of and transfer to the skin of the active ingredient thereby causing an immediate pharmaceutical effect for angina pectoris to be exhibited;

(d) high concentration of ISDN in the blood per unit skin area can be maintained, compared with conventional pharmaceutical preparations, thereby making it possible to cause an excellent pharmaceutical effect with only a small area of application to the skin;

(e) reduction in any unpleasant feeling due to its application to the skin since the pharmaceutical effect can be exhibited with a minimised application area to the skin;

(f) the pharmaceutical preparation can be applied to the skin by a simple operation due to the minimized application area; and

(g) production of red spots on the skin can be minimised due to the special composition of the adhesive material.

For a better understanding of the invention, reference will now be made to the accompanying drawings in which:

Figure 1 is a graphical representation showing the relationship between the application area of a pharmaceutical preparation in tape form and the concentration of ISDN in the blood;

Figure 2 is a graphical representation showing the relationship between the thickness of the adhesive layer of the pharmaceutical preparation in tape form and the transfer of ISDN to the skin; and

Figure 3 is a grapical representation showing the relationship between the amount of ISDN per 10 $cm^2$ of the adhesive layer of the pharmaceutical preparation in tape form and the concentration of ISDN in the blood.

N-vinyl-2-pyrrolidone (VP), the molecular weight of which is 111, has the following structural formula:

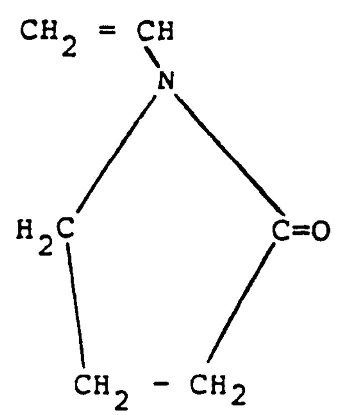

A VP homopolymer, which is water-soluble, meets standard requirements for medical supplies.

The adhesive coating material employed in the present invention is a copolymer of 2-ethylhexyl acrylate (EHA) and N-vinyl-2-pyrrolidone (VP), which satisfies the requirements for an adhesive coating material having properties such as adhesiveness, solubility for ISDN, release of ISDN and reduced skin irritation. More specifically, the copolymer contains EHA in a concentration of 45 mole% or more and VP in a concentration of from 20 to 55 mole%. Preferably the copolymer contains EHA in a concentration of 55 mole% or more and VP in a concentration of 30 to 45 mole%. If VP is less than 20 mole%, ISDN will not be effectively dissolved in the copolymer i.e., the adhesive material. If VP is greater than 55 mole%, the adhesiveness of the adhesive material will be reduced. Even if one or more of the other components such as (meth)acrylate monomer (i.e. methacrylate and/or acrylate monomers) is present in a concentration of 35 mole% or less, more preferably, 15 mole% or less, the desired properties or the abilities of the resulting adhesive material are maintained. A typical (meth)acrylate monomer to be admixed thereto is, in light of the maintenance of excellent adhesiveness, an alkyl (meth)acrylate, for example, propyl acrylate, butyl acrylate, hexyl acrylate, 2-ethyl butyl acrylate, heptyl acrylate, octyl acrylate, nonyl acrylate, decyl methacrylate, or lauryl methacrylate.

The ISDN contained in the adhesive material is present in a concentration of 15% by weight or more of the weight of the adhesive material plus the weight of ISDN, generally 13 to 30% by weight. The ISDN is preferably present in a concentration of 13 to 25% by weight.

When a material dispersed in a compatible state in a matrix diffuses into or outside the matrix, its diffusion rate is generally significantly higher than that of a material existing in a powder or crystal state in the matrix. Thus, to eliminate the conventional drawbacks, it is essential that ISDN should exist within the adhesive material in a compatible state with a concentration which is as close as possible to the saturated solubility concentration thereof. The terminology "the saturated solubility concentration" used herein refers to an ISDN concentration high enough to ensure that the ISDN never crystallises even though the adhesive material containing a certain amount of ISDN is allowed to stand at room temperature for a long period of time. The terminology "a compatible state" used herein refers to a state in which ISDN exists in the saturated concentration or lower within the adhesive material.

According to the invention, although the saturated concentration of ISDN in the adhesive material depends upon the composition of the adhesive material, ISDN should be contained, in a slightly lower concentration that its saturated solubility concentration, in each of the adhesive materials of various compositions.

For maintaining an excellent release level of ISDN, it is necessary that the amount of ISDN to be added to the adhesive material is 80% or more of the saturated solubility concentration of ISDN with respect to the adhesive material. It cannot be said that the adhesive material which is best capable of dissolving ISDN in a

high concentration therein is also the best in release of ISDN therefrom to the skin, since there are unresolved questions with regard to the determination of the distribution coefficient between the adhesive material and the skin. Moreover, there has not been sufficient analysis of the variable phenomena depending upon factors such as perspiration during application of the tape to the skin. However, the adhesive material according to this invention has, in generally, proven superior in transfer of ISDN to the skin.

According to a preferred embodiment of the present invention, a multifunctional monomer is added as a component of the adhesive material to copolymerise with other monomer components, thereby producing slight or extremely slight linkages among the resulting polymers resulting in increased internal cohesive properties of the resulting adhesive material, so that none of the latter will remain on the skin when the tape is removed, regardless of skin conditions or the amount of perspiration on the skin. Furthermore, the use of such a multifunctional monomer as a component of the adhesive material has no effect on the release of the active ingredient and results in reduced irritation of the resulting adhesive material. Examples of suitable multifunctional monomers are di(meth)acrylates obtained by reaction of (meth)acrylic acid with polymethylene glycols such as hexamethylene glycol or octamethylene glycol; di(meth)acrylates obtained by reaction of (meth)acrylic acid with polyalkylene glycols such as polyethylene glycol or polypropylene glycol; tri(meth)acrylates such as trimethylolpropane tri(meth)acrylate and glycerin tri(meth)acrylate; and tetra(meth)acrylates such as pentaerythritol tetra(meth)acrylate. One or more of the multifunctional monomers are added to other monomers to be compolymerised to form the adhesive material, the added amount of which is preferably in a range of 0.005 to 0.5% by weight of the total amount of monomers to be copolymerised. If it is less than 0.005% by weight, the internal cohesive properties due to linkages will not be effectively attained. If it is over 0.5% by weight, the resulting adhesive material will tend to gelatinize resulting in reduced diffusion and/or release of ISDN.

The flexible backing used in this invention, which is not permeable to the active ingredient, i.e. ISDN, may be for example, a single film composed of a polyester such as polyethylene terephthalate, a polyamide such as nylon 6, or a polyurethane; a laminated film composed of such single films; or a laminated film composed of these single films and a polyethylene (PE) film and/or an ethylene-vinyl acetate copolymer (EVA) film.

In general, crystallisation of chemical compounds occurs at the interface between the foreign substances and the matrix containing the chemical compounds therein. In the pharmaceutical preparation according to this invention, although it appears that ISDN might crystallise at the interface between the adhesive layer and the backing due to its structure, however, such crystallisation is not oberved with any of the above-mentioned films when ISDN dissolved in the adhesive material is in a concentration of between 80% (inclusive) and 100% (exclusive) of the saturated solubility concentration thereof. This phenomenon might be due to properties of the adhesive material resulting from the composition as described above.

The percutaneous administration type pharmaceutical preparation in tape form according to this invention can be prepared as follows:

Given amounts of EH and VP, and if required (meth)acrylate monomers and/or multifunctional monomers are admixed with a polymerization solvent such as ethyl acetate, followed by a radical polymerization reaction at about 55°C to 75°C for 8 to 40 hours in a gaseous nitrogen atmosphere, resulting in a polymer generally having a solid content of 15 to 40% by weight, the viscosity of which is generally in the range from 1 to 100 Pa·s 1,000 to 100,000 cPs) at a solid content of 25% by weight, while the molecular weight (average molecular weight calculated in terms of styrene by a gel permeation chromatography) is generally in the range from 100,000 to 1,000,000. The residual EHA and VP monomers in the polymer are generally less than 2% by weight, respectively, of the total weight of solid.

As a polymerization initiator, azobis derivatives or peroxides, for example, may be employed. Examples of suitable azobis derivatives are 2,2'-azobis-isobutyronitrile (AIBN); 1,1'-azobis-cyclohexane-1-carbonitrile; and 2,2'-azobis-2,4-dimethylvaleronitrile. Examples of suitable peroxides are benzoyl peroxide (BPO); lauroyl peroxide (LPO); and di-tertiary butyl peroxide.

To the adhesive material prepared in the above-described manner there is then added an ISDN solution prepared by dissolving ISDN in a suitable solvent such as ethyl acetate, resulting in a coating solution. The coating solution is then applied to either a release paper or a flexible backing, using a coating machine such as for example a direct coater or a reverse coater, in a given thickness, and then dried at a temperature of, for example, 70°C or less to form an adhesive coating layer, generally containing ISDN in a concentration of, for example, from 13 to 30% by weight, the solvent in a concentration of 100 ppm or less and a trace (0.1% by weight or less) of residual monomers and having a ball tack value of 15 or more, on the surface of which a flexible backing or a release paper is then laminated to form the desired percutaneous administration type pharmaceutical preparation in tape form.

The following Examples illustrate the invention.

### Example 1

A separable flask was charged with 317.9 g (70 mole%) of EHA, 82.1 g (30 mole%) of VP and 70.6 g of ethyl acetate to form a monomer solution which is in a monomer concentration of 85% by weight. The solution was heated at 60°C for 32 hours in a nitrogen atmosphere while adding, dropwise thereto, lauroyl peroxide as a polymerization initiator and ethyl acetate as a polymerization solvent. To the resulting

polymer, an ethyl acetate solution of ISDN was added resulting in a coating solution having a solid (total solid of the polymer and the ISDN) content of 25% by weight and an ISDN solid content of 14% by weight. In the same manner as described above, two other kinds of coating solution having a total solid content of 25% by weight and ISDN solid contents of 16 and 18% by weight, respectively, were prepared. Each of these solutions were coated on a release paper having a thickness of 35 µm made of silicone-treated polyethylene terephthalate (PET) and dried to form on the release paper an adhesive base layer having a thickness of 60 µm, on which a backing of PET having a thickness of 9 µm was placed resulting in the pharmaceutical preparation in tape form.

Example 2

In the same manner as in Example 1, 215.2 g (45 mole%) of EHA and 129.7 g (45 mole%) of VP, 55.1 g (10 mole%) of decyl methacrylate and 0.01% by weight (40.0 mg) of trimethylolpropane triacrylate were subjected to polymerization. Using the resulting polymer, four coating solutions having ISDN solid contents of 22, 24, 26 and 28% by weight, respectively, were prepared to obtain four different pharmaceutical preparations.

Example 3

In the same manner as in Example 1, 302.0 g (65 mole%) of EHA, 98.0 g (35 mole%) of VP and 0.02% by weight (80.0 mg) of hexamethyleneglycol dimethacrylate were used to prepare four coating solutions having ISDN solid contents of 14, 16, 18 and 20% by weight, respectively, resulting in four different pharmaceutical preparations.

Example 4

In the same manner as in Example 1, 261.9 g (55 mole%) of EHA, 71.8 g (25 mole%) of VP, 66.3 g (20 mole%) of butyl acrylate and 0.01% by weight (40.0 mg) of polypropylene glycol diacrylate were used to prepare two coating solutions having ISDN solid contents of 14 and 16% by weight, respectively, resulting in two different pharmaceutical preparations.

Control 1

In the same manner as in Example 1, 188.7 g (35 mole%) of EHA and 211.3 g (65 mole%) of VP were used to prepare two coating solutions having ISDN solid contents of 24 and 28% by weight, respectively, resulting in two different pharmaceutical preparations.

Experiment 1

The ISDN-saturated solubilities and the adhesiveness of the pharmaceutical preparations obtained above were examined, the results being shown in the following Table 1.

The ISDN-saturated solubilities were determined as follows:

By peeling off the release paper, the surface of the adhesive layer was exposed and several needle-shaped ISDN crystals were placed thereon. The surface was then covered again by the release paper, enclosed in an aluminium laminated film and maintained at room temperature for one month, after which the growth of ISDN crystals was observed. In Table 1 the symbol "○" indicates that crystal growth was not observed, the symbol "△" indicates that crystal growth was indefinite, and the symbol "X" indicates that crystal growth was definitely observed. The ISDN-saturated solubilities of each of the pharmaceutical preparations in Examples 1 to 4 and Control 1 are given in parentheses in Table 1.

The adhesiveness was examined by a ball tack method at room temperature with respect to each of the pharmaceutical preparations just after their manufacture. The symbol "○" indicates excellent adhesiveness and the symbol "X" indicates an inferior adhesiveness.

TABLE 1

| | ISDN Solid Contents (wt%) | Crystal Growth | Adhesiveness |
|---|---|---|---|
| Example 1 | 14 | △ | ○ |
| | 16 | X | ○ |
| | 18 | X | ○ |
| Example 2 | 22 | ○(26) | ○ |
| | 24 | ○ | ○ |
| | 26 | △ | ○ |
| | 28 | △ | ○ |
| Example 3 | 14 | ○(17) | ○ |
| | 16 | ○ | ○ |
| | 18 | X | ○ |
| | 20 | X | ○ |
| Example 4 | 14 | X | ○ |
| | 16 | X | ○ |
| Control 1 | 24 | ○(34) | X |
| | 28 | ○ | X |

Table 1 indicates that the pharmaceutical preparation having the composition in Control 1 was inferior in adhesiveness.

Next, using the two different adhesive base materials described in Examples 2 and 3, the six different pharmaceutical preparations, as shown in Table 2, having an ISDN concentration of 90% of the ISDN-saturated solubilities which are given in parentheses in Table 1, were prepared in the same manner as in Example 1. Three sample preparations in each Example were cut into squares, the first of which had an area of 10 $cm^2$ with a thickness of 60 μm, the second of which had a thickness of 60 μm and contained ISDN of 10 mg therein, and the third of which had an area of 10 $cm^2$ and contained ISDN of 10 mg therein.

TABLE 2

| Composition | Sample No. | ISDN Concentration (wt%) | Thickness of Base Layer (µm) | Area ($cm^2$) | ISDN Content (mg) |
|---|---|---|---|---|---|
| | 2—1 | | 60 | 10 | 14.0 |
| Example 2 | 2—2 | 23.4 | 60 | 7.1 | 10 |
| | 2—3 | | 43 | 10 | 10 |
| | 3—1 | | 60 | 10 | 9.2 |
| Example 3 | 3—2 | 15.3 | 60 | 10.9 | 10 |
| | 3—3 | | 65 | 10 | 10 |

Table 2 indicates that the required surface area of each of the preparations according to this invention is extremely small when sample preparations of the same ISDN content and the same thickness of the adhesive materials are prepared.

Using these preparations, the following Experiments 2 to 5 were carried out.

Experiment 2

Each of the two different pharmaceutical preparations (Sample Nos. 2—2 and 3—2) containing ISDN of 10 mg was applied to the back of a rabbit (Japanese White sp.) on a portion where the hair had been removed. After a predetermined period of time (i.e., 1, 4, 8 and 24 hours), a certain amount of blood was collected and subjected to the determination of the ISDN concentration therein. This experiment was repeated three times. The results are shown in Figure 1 indicating that the sample preparations according to this invention are in approximately the same concentration of ISDN in the blood.

Experiment 3

Each of the two pharmaceutical preparations (Sample Nos. 2—3 and 3—3) having an area of 10 $cm^2$ and an ISDN content of 10 mg was applied to the back and the side of a rabbit (Japanese White sp.) on each of the portions where the hair had been removed. After a predetermined period of time (i.e. 2, 8 and 24 hours), each of the preparations were removed and subjected to an ISDN extraction treatment with methanol to extract ISDN, and then measured by liquid chromatography. The determination values were subtracted from 10 mg of the initial ISDN contents, resulting in the transfer values of ISDN to the skin. The determination of the transfer of ISDN to the skin was repeated three times. The results are shown in Figure 2.

Experiment 4

Each of the two pharmaceutical preparations (Sample Nos. 2—1 and 3—1), having an area of 10 $cm^2$ and a base layer thickness of 60 µm, and a 10 $cm^2$ piece of Blenderm (Trade Mark) surgical tape were applied to a rabbit in the same manner as in Experiment 3. After 24 hours, all of the tapes were removed to determine irritation to the skin and the residual amount of the adhesive base material. These procedures were repeated four times.

The irritation test was carried out by evaluating the intensity of the red spots on the skin just after removal of the tapes and at 48 hours thereafter. The evaluation marks are as follows: the mark zero (0), indicates that red spots were not observed at all, the mark 1 indicates that traces of red spot were observed, the mark 2 indicates that traces of red spots were clearly observed, the mark 3 indicates that slightly cardinal red spots were observed, and the mark 4 indicates that cardinal red spots were observed. In this experiment, neither incrustations nor edemata were observed on the skin.

The total evaluation marks were averaged to represent the irritation indexes of each of the evaluated tapes and shown in Table 3.

TABLE 3

| Tape | Example 2 | Example 3 | Surgical Tape |
|---|---|---|---|
| Index of Irritation to the skin | 1.50 | 1.25 | 1.25 |

Table 3 indicates that the pharmaceutical preparations according to this invention result in extremely reduced irritation to the skin because their irritation indexes are approximately equal to those of the Blenderm surgical tape.

A test of the residual amount of adhesive was carried out just after removal of the tapes. The evaluation marks are as follows: the mark zero (0) indicates that the residual amount was zero, the mark 1 indicates that the residual amount was slight, the mark 2 indicates that the residual adhesive was observed on the skin corresponding to the corners and/or the edges of the tape, and the mark 3 indicates that the residual adhesive was observed on the skin correspond to a half or more portion of the tape.

The total evaluation marks were averaged to represent the residual adhesive indexes of each of the evaluated tapes and shown in Table 4.

TABLE 4

| Tape | Example 2 | Example 3 | Surgical Tape |
|---|---|---|---|
| Index of Residual Adhesive | 0 | 0 | 0.50 |

Table 4 indicates that the pharmaceutical preparations according to this invention exhibit excellent adhesiveness and reduced residual amount of the adhesive.

Experiment 5

Each of the two pharmaceutical preparations (Samples Nos. 2—1 and 3—1) (all having the same thickness but varying in ISDN content) was applied to the back of a rabbit on a portion where the hair had been removed. After a predetermined period of time (i.e. 1, 6 and 24 hours), the concentration of ISDN in the blood was measured, the results of which are shown in Figure 3. The determination was repeated three times.

Figure 3 shows that the pharmaceutical preparations according to this invention are capable of giving an increased concentration of ISDN in the blood.

**Claims**

1. A percutaneously administrable pharmaceutical preparation in the form of an adhesive tape comprising a flexible backing which is not permeable to the pharmaceutically active ingredient, and an adhesive coating layer on said flexible backing, the adhesive layer comprising an adhesive material and a pharmaceutically active ingredient compatible with said adhesive material, characterised in that said adhesive material is a copolymer derived from 2-ethylhexyl acrylate (EHA) in a concentration of 45 to 80 mole%, at least one (meth) acrylate monomer in a concentration of from 0 to 35 mole%, the monomer being selected from propyl acrylate, butyl acrylate, hexyl acrylate, 2-ethyl butyl acrylate, heptyl acrylate, octyl acrylate, nonyl acrylate, decyl methacrylate and lauryl methacrylate, and N-vinyl-2-pyrrolidone (VP) in a concentration of from 20 to 55 mole%, with the sum of the mole% of EHA, (meth); acrylate and VP being 100, and said pharmaceutically active ingredient is isosorbide dinitrate (ISDN) which is present in said adhesive material in a concentration of 13 to 30% by weight and which is at least 80% of the saturated solubility concentration of ISDN in said adhesive material.

2. A pharmaceutical preparation as claimed in Claim 1, wherein the adhesive material is a copolymer derived from EHA in a concentration of 55 to 70 mole%, a (meth) acrylate monomer in a concentration of 0 to 15 mole% and VP in a concentration of 30 to 45 mole%, and the concentration of ISDN in said adhesive material is 13 to 25% by weight.

3. A pharmaceutical preparation as claimed in Claim 1 or 2, wherein the adhesive material is a copolymer further derived from at least one multifunctional monomer in a concentration of 0.005 to 0.5%

by weight of the total amount of monomers therein, the multifunctional monomer being selected from di(meth) acrylates, tri(meth) acrylates and tetra(meth) acrylates.

4. A pharmaceutical preparation as claimed in Claim 1, 2 or 3, wherein the adhesive material is a copolymer containing no (meth) acrylate monomer.

5. A pharmaceutical preparation as claimed in Claim 3, wherein the multifunctional monomer is selected from di(meth) acrylates.

6. A pharmaceutical preparation as claimed in Claim 5, wherein the di(meth) acrylate is obtained by the reaction of (meth) acrylic acid with a polymethylene glycol or by the reaction of (meth) acrylic acid with a polyalkylene glycol.

7. A pharmaceutical preparation as claimed in any preceding claim, wherein the flexible backing is formed from a film of polyethylene terephthalate.

8. A pharmaceutical preparation as claimed in any preceding claim, wherein the adhesive layer is covered with a release paper.

**Patentansprüche**

1. Perkutan zu verabreichendes Arzneimittelpräparat in der Form, eines Heftpflasters, umfassend eine flexible Unterlage, die für den pharmazeutischen Wirkstoff undurchlässig ist, und eine klebrige Beschichtung auf der flexibilen Unterlage, wobei klebrige Schicht ein klebriges Material und einen pharmazeutischen Wirkstoff umfaßt, der mit der klebrigen Material verträglich ist, dadurch gekennzeichnet, daß das klebrige Material ein Copolymer ist, abgeleitet von 2-Ethylhexylacrylat (EHA) in einer Konzentration von 45 bis 80 Mol%, wobei mindestens ein (Meth)acrylat-Monomer in einer Konzentration von 0 bis 35 Mol% vorliegt und das Monomer aus Propylacrylat, Butylacrylat, Hexylacrylat, 2-Ethylbutylacrylat, Heptylacrylat, Octylacrylat, Nonylacrylat, Decylmethacylat und Laurylmethacrylat ausgewählt ist, und N-Vinyl-2-pyrrolidon (VP) in einer Konzentration von 20 bis 55 Mol%, wobei die Summe der Mol% von EHA, (Meth)acrylat und VP 100 ist, und der pharmazeutische Wirkstoff Isosorbid-Dinitrat (ISDN) ist, das in dem klebrigen Material in einer Konzentration von 13 bis 30 Gew.% vorliegt und mindestens 80% der Konzentration bei gesättigter Lösung von ISDN in dem klebrigen Material ausmacht.

2. Arzneimittelpräparat nach Anspruch 1, in dem das klebrige Material ein Copolymer ist, abgeleitet von EHA in einer Konzentration von 55 bis 70 Mol%, einem (Meth)acrylat-Monomer in einer Konzentration von 0 bis 15 Mol% und VP in einer Konzentration von 30 bis 45 Mol%, und die Konzentration von ISDN in dem klebrigen Material 13 bis 25 Gew.% beträgt.

3. Arzneimittelpräparat nach Anspruch 1 oder 2, in dem das klebrige Material ein Copolymer ist, das sich ferner ableitet von mindestens einem multifunktionellen Monomer in einer Konzentration von 0,005 bis 0,5 Gew.% der Gesamtmenge an darin enthaltenen Monomeren, wobei das multifunktionelle Monomer aus Di(meth)acrylaten, Tri(meth)acrylaten und Tetra(meth)acrylaten ausgewählt ist.

4. Arzneimittelpräparat nach Ansprüchen 1, 2 oder 3, in dem das klebrige Material ein Copolymer ist, das kein (Meth)acrylat-Monomer enthält.

5. Arzneimittelpräparat nach Anspruch 3, in dem das multifunktionelle Monomer aus Di(meth)acrylaten ausgewählt ist.

6. Arzneimittelpräparat nach Anspruch 5, in dem das Di(meth)acrylat erhalten wird, indem (Meth)-acrylsäure mit einem Polymethylenglykol umgesetzt wird oder (Meth)acrylsäure mit einem Polyalkylenglykol umgesetzt wird.

7. Arzneimittelpräparat nach einem der vorhergehenden Ansprüche, in dem die flexible Unterlage aus einem Film von Polyethylenterephthalat gebildet wird.

8. Arzneimittelpräparat nach einem der vorhergehenden Ansprüche, in dem die klebrige Schicht mit einem abziehbaren Papier bedeckt ist.

**Revendications**

1. Préparation pharmaceutique administrable par voie percutanée, sous forme d'un ruban adhésif comportant un support flexible qui n'est pas perméable à l'ingrédient pharmaceutiquement actif, ainsi qu'une couche de revêtement adhésif sur ledit support flexible, la couche adhésive comprenant un matériau adhésif et un ingrédient pharmaceutiquement actif compatible avec ledit matériau adhésif, caractérisée en ce que ledit matériau adhésif est un copolymère dérivé de l'acrylate de 2-éthylhéxyle (EHA) dans une concentration de 45 à 80 moles%, d'au moins un monomère de (méth)acrylate dans une concentration de 0 à 35 moles%, le monomère étant choisi parmi les composés suivants acrylate de propyle, acrylate de butyle, acrylate d'héxyle, acrylate de 2-éthylbutyle, acrylate d'heptyle, acrylate d'octyle, acrylate de nonyie, méthacylate de décyle, et méthacrylate de lauryle et de la N-vinyl-2-pyrrolidone (VP) dans une concentration de 20 à 55 moles%, le total des moles% de EHA, (méth)acrylate et VP étant 100, et en ce que ledit ingrédient pharmaceutiquement actif est le dinitrate d'isosorbide (ISDN) qui est présent dans ledit matériau adhésif dans une concentration de 13 à 30% en poids et qui est au moins 80% de la concentration d'ISDN en solution saturée dans ledit matériau adhésif.

2. Préparation pharmaceutique selon la revendication 1, dans laquelle le matériau adhésif est un copolymère dérivé du EHA dans une concentration de 55 à 70 moles%, d'un monomère du (méth)acrylate

dans une concentration de 0 à 15 moles% et de la VP dans une concentration de 30 à 45 moles%, et dans laquelle la concentration du ISDN dans ledit matériau adhésif est de 13 à 25% en poids.

3. Préparation pharmaceutique selon la revendication 1 ou 2, dans laquelle le matériau adhésif est un copolymère dérivé en outre d'au moins un monomère multifonctionnel dans une concentration de 0,005 à 0,5% en poids de la quantité totale de monomères qui s'y trouvent, le monomère multifonctionnel étant choisi parmi les composés di(méth)acrylates, tri(meth)acrylates et tétra(méth)acrylates.

4. Préparation pharmaceutique selon la revendication 1, 2 ou 3, dans laquelle le matériau adhésif est un copolymère ne contenant pas de monomère du (méth)acrylate.

5. Préparation pharmaceutique selon la revendication 3, dans laquelle le monomère multifonctionnel est choisi parmi les di(méth)acrylates.

6. Préparation pharmaceutique selon la revendication 5, dans laquelle on obtient le di(méthy)acrylate par la réaction de l'acide (méth) acrylique avec un polyméthylèneglycol ou par la réaction de l'acide (méth) acrylique avec un polyalcoylèneglycol.

7. Preparation pharmaceutique selon l'une quelconque des revendications précédentes, dans laquelle le support flexible est formé d'un film de téréphthalate de polyéthylène.

8. Preparation pharmaceutique selon l'une quelconque des revendications précédentes, dans laquelle la couche adhésive est recouverte d'un papier à enlever.

FIG. 1

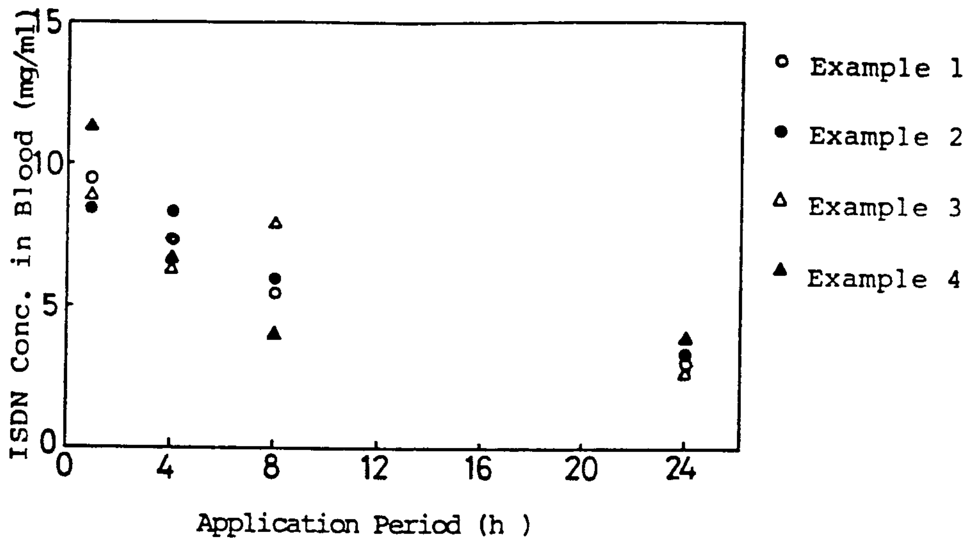
ISDN Conc. in Blood (mg/ml)
15
10
5
0
0
4
8
12
16
20
24
Application Period (h )
Example 1
Example 2
Example 3
Example 4

FIG. 2

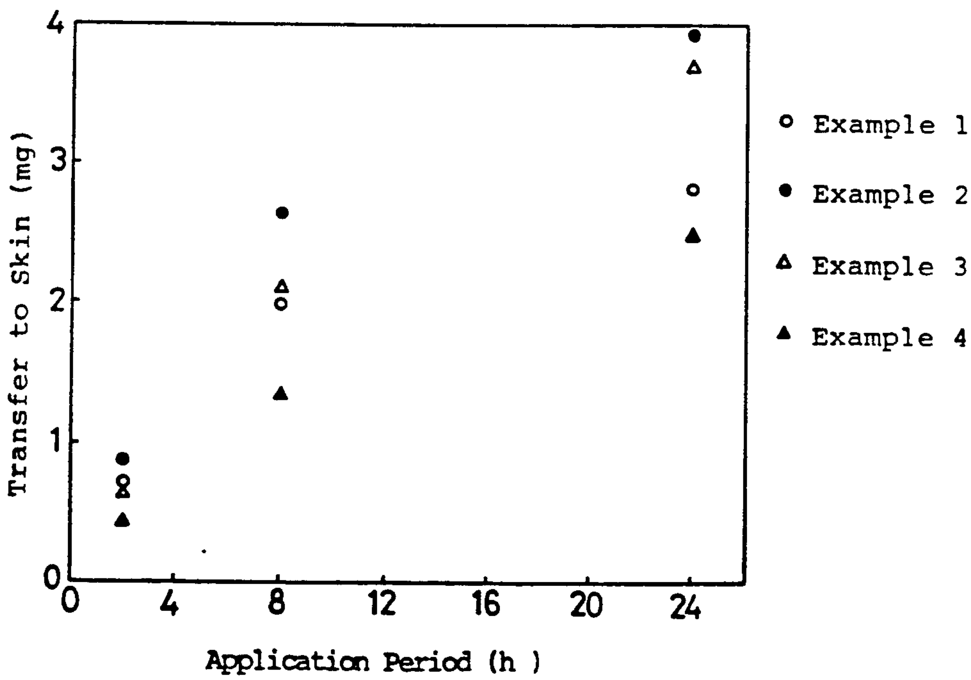
Transfer to Skin (mg)
4
3
2
1
0
0
4
8
12
16
20
24
Application Period (h )
Example 1
Example 2
Example 3
Example 4

FIG. 3

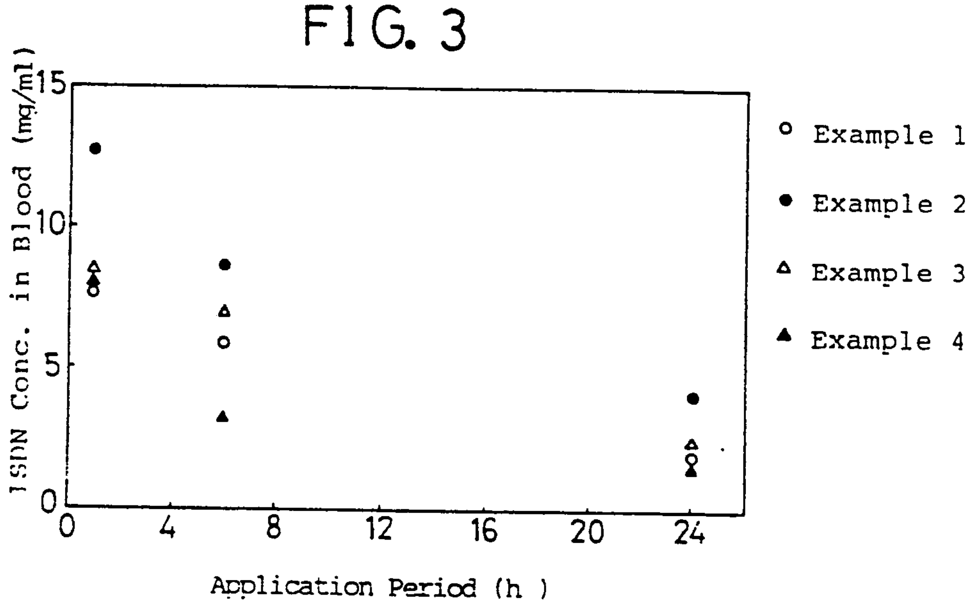
ISDN Conc. in Blood (mg/ml)
15
10
5
0
0
4
8
12
16
20
24
Application Period (h )
Example 1
Example 2
Example 3
Example 4